(19) Europäisches Patentamt
European Patent Office
Office européen des brevets

(11) **EP 2 329 878 A1**

(12) **EUROPEAN PATENT APPLICATION**
published in accordance with Art. 153(4) EPC

(43) Date of publication:
08.06.2011 Bulletin 2011/23

(51) Int Cl.:
*B01J 23/34* (2006.01)   *B01J 23/889* (2006.01)

(21) Application number: 08876993.0

(22) Date of filing: 22.09.2008

(86) International application number:
PCT/JP2008/067134

(87) International publication number:
WO 2010/032338 (25.03.2010 Gazette 2010/12)

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(71) Applicants:
• Waseda University
Tokyo 169-8050 (JP)
• Sued-Chemie Catalysts Japan, Inc.
Shibuya-ku
Tokyo 151-0053 (JP)

(72) Inventors:
• KIKUCHI, Eiichi
Tokyo 169-8555 (JP)
• SEKINE, Yasushi
Tokyo 169-8555 (JP)
• MISHIMA, Yuji
Toyama-shi
Toyama 939-2753 (JP)

(74) Representative: Baronetzky, Klaus
Splanemann
Patentanwälte Rechtsanwalt Partnerschaft
Rumfordstrasse 7
80469 München (DE)

(54) **DEHYDROGENATION CATALYST FOR ALKYL AROMATIC COMPOUNDS HAVING HIGH REDOX CATALYSIS, PROCESS FOR PREPARATION OF THE CATALYST AND PROCESS OF DEHYDROGENATION WITH THE SAME**

(57) The object of the present invention is to provide the catalyst used in a process for preparation of alkenylaromatic compounds by dehydrogenating alkyl aromatic compounds by means of steam as a diluent, wherein the catalyst prevents the block and corrosion caused by the alkali metal component migrated from the catalyst, and the process for producing same, and the dehydrogenation method using it.

The solid catalyst which has the high redox ability and comprises no alkali metal component migrated from the catalyst in the presence of steam with high temperature is used.

[Fig.3]

Reaction Condition
Cat. Wt = 1.2 g
WHSV = 1.2 $g_{EB}$ $g_{cat}^{-1}$ $h^{-1}$
$N_2$/ EB / $H_2O$ (mol ratio) = 3 / 1 / 12

Ethylbenzene Conversion / %
Temperature / K

◇ Example 1 ($La_{0.8}Ba_{0.2}MnO_{3-\delta}$)
△ Example 2 ($La_{0.8}Ba_{0.2}Mn_{0.6}Fe_{0.4}O_{3-\delta}$)
● Comparative Example 1(Styromax®-4)

EP 2 329 878 A1

**Description**

[Field of the Invention]

The present invention relates to a catalyst used in a process for preparation of alkenylaromatic compounds by means of dehydrogenation by contacting alkylaromatic compounds diluted with steam with the catalyst, a process for producing same, and a dehydorogenation method using same.

[Description of the Related Art]

A styrene monomer (SM) which is the alkenylaromatic compound is an important industrial material which is widely used as a raw material for polystyrene, an ABS resin or a synthetic rubber. It is industrially produced by means of the dehydrogenation by mainly contacting ethylbenzene (EB) with a catalyst under a diluted condition with steam, where a simple dehydrogenation reaction is carried out as [Formula 1]:

$$[\text{Formula 1}] \qquad EB \rightarrow SM + H_2$$

The ethylbenzene dehydrogenation reaction is an endothermic reaction of about 30 kcal per 1 mole, and is an equilibration reaction of the molecule increase in which a total 2 mole of a product which contains 1 mole of styrene monomer and 1 mole of hydrogen is obtained from 1 mole of an ethylbenzene material. Thus, to obtain a high single current yield, preference is given to a high reaction temperature and a low ethylbenzene partial pressure. In producing the styrene monomer on an industrial scale, it is usually carried out at a reaction temperature of 500 to 650 °C in the presence of a diluted steam. A role of steam is, for example, a decreasing of the ethylbenzene partial pressure, as well as a heat supply for retaining the reaction temperature, a suppression of a carbonaceous material accumulated on the catalyst and a prevention of a catalyst deactivation by hindering a reduction of the catalyst. For these reasons, a large amount of steam is industrially used in the ethylbenzene dehydrogenation reaction, and its flow rate generally ranges from 5 to 15 mol/mol at a molar ratio of the steam flow rate to the ethylbenzene monomer material.

In producing the styrene monomer by means of the ethylbenzene simple dehydrogenation, a catalyst which basically comprises an iron oxide and potassium is widely used as described in the Non-Patent Document 1. As described in the Non-Patent Document 2, the catalyst which comprises the iron oxide and potassium as main components forms a potassium ferrate ($KFeO_2$) phase as a metastable phase in the reaction, and promotes the simple dehydrogenation reaction as is an active species. As a catalyst for the ethylbenzene dehydrogenation reaction under the diluted steam, it has proposed of the catalyst comprising the iron oxide and potassium as main components as described in, for example, the Patent Document 1, Patent Document 2 and Patent Document 3. As indicated in these Patent Documents, in the catalyst for the ethylbenzene dehydrogenation reaction under the diluted steam, a highly active site $KFeO_2$ with a high reaction rate is constituted, and thus it is considered that the iron oxide-potassium component is necessarily used.

The ethylbenzene dehydrogenation reaction is widely industrially carried out under the diluted steam by means of the catalyst which basically comprises the iron oxide-potassium, but in the case that a long-time operation is carried out on the industrial scale, there are problems as follows. That is, as described in the Non-Patent Document 3, the catalyst comprising the iron oxide-potassium forms a KOH species with a high vapor pressure by partially reacting the potassium species in the catalyst with steam present in the reaction system, and thus the potassium species migrate from the catalyst layer near a reaction gas inlet of the high temperature area. The migrate potassium species deposits as a potassium compound at a lower temperature position than a reactor such as a heat exchanger located at downstream of the reactor, then a blocking takes places and the pressure in the reactor system raises and the styrene yield decreases. Alternatively, it brings about a corrosion of the equipment. Also, with regard to a productivity improvement, there is an industrial problem in point of that a cleaning must be carried out during a reaction stop due to removing potassium accumulated in the equipment.

To stably produce the styrene monomer on industrial scale without the problems as mentioned-above, it is desired to provide the catalyst without scattering potassium. For prevention of potassium migration, preference is given to the catalyst having no potassium as component, but it has mostly not proposed of the catalyst wholly having no potassium, which is used in the ethylbenzene dehydrogenation reaction under the diluted steam. As described in the Patent Document 4, only catalyst with a perovskite structure which in particular comprises La-Sr-Fe-CoO$_3$ as component is proposed. However, the Patent Document 4 only discloses the perovskite structure, and an important point with reference to a chemical property of the catalyst having no potassium is not indicated. Therefore, it has not entirely shown that which catalyst can be a useful catalyst. Also, the performance of the proposed catalyst is extremely lower than an ethylbenzene conversion obtained by means of the catalyst which is widely used and comprises the iron oxide-potassium, and thus it is insufficient to industrially use.

Patent Document 1: Patent Number 3881376
Patent Document 2: Patent Number 2833907
Patent Document 3: Patent Number 2604426
Patent Document 4: WO-A 99/64377
Non Patent Document 1: Catalyst, vol. 33, no.1, p.9-14 (1991)
Non Patent Document 2: Applied Catalysis, viol.26, p.81 (1986)
Non Patent Document 3: Journal of Catalysis, vol.126, p.339 (1990)

[Summary of the Invention]

Problems to be Resolved by the Invention

To solve the above-mentioned disadvantage in the related art, it is the object of the present invention to provide a catalyst which if the alkenylaromatic compounds are stably produced on industrial scale, prevents the potassium migration, reduces the operation problem related to the catalyst, and improves the activity without losing the selectivity of the catalyst.

Means of Solving the Problems

We have diligently investigated and surprisingly found that in the dehydrogenation reaction by contacting an alkylaromatic compound such as ethylbenzene diluted with steam with the catalyst, the dehydrogenation reaction proceeds by means of the simple dehydrogenation reaction represented by [Formula 1] generally suggested, as well as the redox reaction obtained by combining two reactions, i.e. the oxidative dehydrogenation reaction by reacting the catalytic lattice oxygen wtih the ethylbenzene material as represented in [Formula 2] and the reoxidizing reaction of the lattice oxygen defect by steam as represented in [Formula 3]. We can found that if the redox reaction is quick enough, it is not necessary to use the potassium ferrate phase which is the active substance by the simple dehydrogenation reaction in the related art, and the alkenyl compound, such as ethylbenzene can be produced.

[Formula 2]        $EB + (\text{Catalytic Lattice Oxygen}) \rightarrow SM + H_2O + (\text{Lattice Oxygen Defect in the Catalyst})$

[Formula 3]        $(\text{Lattice Oxygen Defect in the Catalyst}) + H_2O \rightarrow (\text{Catalytic Lattice Oxygen}) + H_2$

In the properties of the catalyst for effectively and continuously holding the combined reaction by the [Formula 2] and the [Formula 3], it is necessary to possess a high ability wherein the lattice oxygen present in the catalyst can freely go in and out, i.e. a high redox ability. The redox ability is defined as a ratio of the amount of oxygen utilizable for redox reaction to all lattice oxygen in the catalyst, and its amount can be determined by the redox transient response method, for example, in which each of ethylbenzene and steam under a noble gas are periodically and alternatively passed through the catalyst. Contacting the catalyst with the ethylbenzene vapor diluted with the noble gas brings about the reaction indicated in [Formula 2], and a part of the lattice oxygen in the catalyst is used in the oxidative dehydrogenation reaction and is discharged, resulting in producing $H_2O$. Also, after contacting the ethylbenzene vapor, as represented in [Formula 3], contacting steam diluted with the noble gas with the catalyst brings about producing $H_2$ by regenerating the lattice oxygen defect in the catalyst by means of steam. Determining the amount of $H_2O$ obtained by contacting the ethylbenzene vapor, or the amount of $H_2$ obtained by contacting steam after contacting the ethylbenzene vapor, makes it possible to measure the amount of lattice oxygen utilizable for redox reaction present in the catalyst.

In the catalyst suitable for the dehydrogenation catalyst of alkyl aromatic compounds under the diluted steam, it is important to have greater than or equal to 2.3 % of ratio based on the amount of the lattice oxygen utilizable for redox reaction by the above-mentioned measurement and the calculated value of all lattice oxygen in the catalyst calculated by the following [Formula 4] in consideration of the composition in the catalyst. Particularly preference is given to a value of 2.4 to 50%.

[Formula 4]

$$\text{The amount of all lattice oxygen in the catalyst } (\mu mol \bullet g_{-cat}^{-1})$$
$$= 3 \times 10^6 \div \text{formula weight of composition of each catalysts } (g \cdot mol^{-1})$$

Therefore, the present invention provides the dehydrogenation catalyst of alkyl aromatic compounds for effectively and sophisticatedly producing the alkenyl aromatic compounds by activating the lattice oxygen in the catalyst by means of steam, a process for producing same, and a dehydrogenation method using it.

Effect of the Invention

The catalyst according to the invention makes it possible to obtain the catalyst having the high redox ability as the catalyst for producing alkenyl aromatic compounds by means of dehydrogenation by contacting alkyl aromatic compounds diluted with steam with the catalyst. Thus, it is possible to provide the catalyst which has the same yield as the catalyst containing the conventionally necessary alkali metal component and which considerably improves the operation problems such as block and corrosion at the position lying downstream of the reactor, and the process for producing same, and the dehydrogenation method using it.

[Brief Description of the Drawings]

[Fig.1] is a schematic drawing showing the evaluation device of the catalytic performance for the ethylbenzene dehydrogenation reaction.
[Fig.2] is a schematic drawing showing the device which determines the lattice oxygen atomic weight utilizable for the redox reaction in the catalyst.
[Fig.3] is a drawing showing the ethylbenzene conversion of the catalysts in Example 1, Example 2 and Comparative Example 1 as a function of reaction condition.
[Fig.4] is a drawing showing the ethylbenzene conversion of the catalysts in Comparative Example 2 and Example 1 as a function of reaction condition.
[Fig.5] is a drawing showing the ethylbenzene conversion of the catalysts in Example 3 and Comparative Example 3 as a function of reaction condition.
[Fig.6] is a drawing showing the ethylbenzene conversion of the catalysts in Comparative Example 4 and Comparative Example 5 as a function of reaction condition.

Description of Signs

1      Fixed-Bed Reactor
2      Catalyst
3      Silica Wool
4      Thermocouple
5      Electrical Furnace
6      Ethylbenzene Bubbuler
7      $H_2O$ Pump
8      Ice-cooling Trap
10     Measurement Device for Redox Transient Response
11     Catalyst
12     Silica Wool
13     Thermocouple
14     Ethylbenzene Bubbuler
15     $H_2O$ Bubbuler
16     He Purge Gas
17     Quadrupole Mass Spectrometer
18     Trap

[Best Mode for Practicing the Invention]

The catalyst according to the invention is a catalyst comprising a mixed metal oxide containing rare-earth metal, alkaline-earth metal and transition metal as an essential element, but not containing alkali metal. In consideration of the catalytic activity, it is important to be the catalyst which has the high redox ability and which contains any one of La or Ce as rare-earth metal, one or more elements selected from the group consisting of Mg, Ca, Sr, Ba as alkaline-earth metals and one or more elements selected from the group consisting of Ti, Cr, Mn, Fe, Co, Ni, Cu as transition metals. In the suitable composition proportion of rare-earth metals, alkali-earth metals and transition metals, it is desired to be 0 to 5 based on (mole of alkali-earth metals)÷(mole of rare-earth metals) and 0.5 to 2 based on (mole of alkali-earth metals + mole of rare-earth metals)÷(mole of transition metals). In particular, the catalyst containing elements selected from La, Ba, Mn and Fe and having the composition represented by $La_xBa_{1-x}Mn_{1-y}Fe_yO_{3-\delta}$ (0.2<x<1, y is not less than 0 and not more than 1) has the highest redox ability, and thus gives a high yield of the styrene monomer. The mixed metal oxide has a spinel structure or a perovskite structure. The spinel structure is a crystalline structure represented by a chemical formula $AB_2X_4$ (A, B are a positive element, and X is a negative element.), and the perovskite structure is a structure represented by chemical formula $ABX_3$. In these formulae, the anion X is arranged as a cubic closet-packed structure, and has the structure in which the position of a pair of the face centers can be replaced with the cation A with the same size as X, and the A is coordinated to the 12 Xs. Also, in the structure represented by the $AB_2X_4$ type or the $ABX_3$ type, the elements A and B may be partially replaced with a different element. For example, the structure represented by $A_{1-m}A'_mB_{2n-2}B'_{2n}X_4$ or $A_{1-m}A'_1B_{1-n}B_nX_3$ can be used. Also, the alkali metal not involving the catalyst of the invention is lithium, sodium, potassium, rubidium and cesium. Due to this, various problems such as migration derived from the alkali metal are resolved.

The dehydrogenation catalyst containing the mixed metal oxide showing such high yield of the styrene monomer can be prepared in a known method. For example, it is possible to use a polymerized complex method in which the complex of the component metal is used or a sintering method in which the calcination is carried out after mixing a compound of the component metal as raw material. But, other methods can be used, so far as the high redox ability can be retained. For example, in the sintering method in which the calcination is carried out after mixing a compound of the component metal as raw material, it is necessary for an amount of the added water in the kneading to adapt to the amount in the subsequent extruding. Its amount, depending on the kind of the material used, is usually 1 to 50 % by weight, and after sufficiently kneading it, it is extruded and then dried and sintered to obtain a certain dehydrogenation catalyst pellet. In the dry, it is an enough to remove free water possessed in the extrusion, and it is carried out at the temperature of 80 to 300 °C, preferably 100 to 200°C. On the other hand, the calcination is carried out to thermally decompose each catalyst precursors possessed in a dry substance, to improve the physical stability of the catalyst pellet and to improve its performance, and is carried out at the temperature of 600 to 1400°C, preferably 800 to 1200°C.

If the catalyst according to the invention can be used as a usual fixed-bed catalyst, it may have any shapes, for example, powder, cylindrical, round, star, or may be supported on a carrier.

Also, the dehydrogenation catalyst of alkyl aromatic compounds according to the invention is useful as the dehydrogenation catalyst for producing alkenyl aromatic compounds by contacting alkyl aromatic compounds with steam, in particular, is useful for promoting the dehydrogenation of ethylbenzene if contacting the ethylbenzene with steam is carried out to produce styrene, and physically stabilizes the dehydrogenation reaction in the presence of steam. In the reaction condition of ethylbenzen dehydrogenation reaction if the catalyst with high redox ability is used, a mixing ratio of steam to a total flow rate of ethylbenzen and styrene is not limited, but is usually from 1 to 30 of molar ratio, preferably 5 to 15. If the molar ratio of steam is too low, a coking takes place, and thus it is not possible to obtain an expected ethylbenzen conversion. Also, if the molar ratio of steam is too greater, a large amount of steam which uses much energy in the production must be used, and thus it is not desired in perspective of the industrial efficiency. The reaction temperature of the catalyst layer with which the material having such composition as a gas is contacted is usually not less than 500 °C and not more than 700 °C. If the temperature is less than 500 °C, the obtained ethylbenzene conversion is too low, and thus the styrene monomer cannot be efficiently produced. Also, if the temperature is more than 700 °C, the styrene monomer once produced gives rise to a polymerization or decomposition, resulting in much by-product, and thus it is not industrially desired.

The present invention was explained in details based on the Examples and Comparative Examples as mentioned below. But, the invention is not limited to these Examples and Comparative Examples.

[Example]

[Example 1]

Catalyst Preparation

7.17 g of La(NO$_3$)$_3$•6H$_2$O, 1.093 g of Ba(NO$_3$)$_2$ and 6.063 g of Mn(NO$_3$)$_3$•6H$_2$O are each weighed by means of an electrical weighing machine, and are dissolved in 50 ml of distilled water. On the other hand, citric acid monohydrate and ethylene glycol corresponding to 3 molar equivalence of mole of all metals are dissolved in distilled water, and it is dissolved in the prior metal salt-dissolving solution. The mixture solution is evaporated in a water bath at 353 K for about 16 hours until viscosity arises, then is transported on a hot-stirrer and stirred with heating to about 573 K to completely dry it. The obtained solid is comminuted, is pre-sintered at 673 K for an hour in a muffle furnace, and then is sintered at 1123 K for 10 hours. The obtained sinter is comminuted and then granulated to 0.50 to 0.71 mm of a particle size by means of a sieve. This gives the catalyst with the composition of La$_{0.8}$Ba$_{0.2}$MnO$_{3-\delta}$.

Activity Test

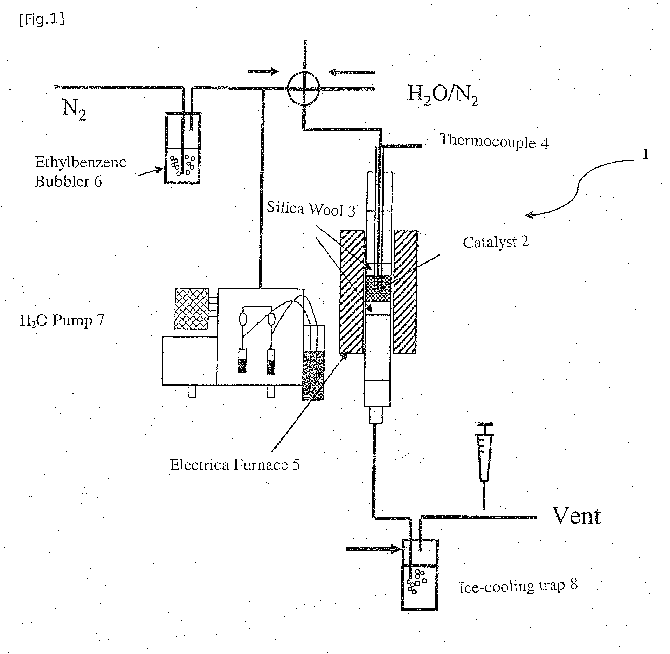

The ethylbenzene dehydrogenation ability of the obtained catalyst 2 is measured by a fix-bed circulation reactor 1 as shown in Fig.1. A quartz tube with 10mm of an external diameter is used as a reaction tube, and 1.00 g of the catalyst is fixed by arrange a silica wool 3 above and under it. To control a reaction temperature, a thermocouple 4 in which a thermowell of the quartz tube with 10mm of an external diameter is inserted into a center of the catalyst layer and an electrical furnace 5 around the quartz tube are disposed. Also, ethylbenzene is fed by means of an ethylbenzene bubbler 6, and the activity test is carried out by means of a H$_2$O pump 7 under the following condition.
Weigh Hourly Space Velocity (WHSV) = 1g$_{-ethylbenzene}$•g$_{-cat}^{-1}$•h$^{-1}$
H$_2$O Flow Rate/ Ethylbenzene Flow Rate/ N$_2$ Flow Rate =12 mol/ 1 mol/ 3 mol
Reaction Temperature = 783K, 813K, 843K, 873K, 913K
Pressure = atmospheric Pressure
Products obtained at each reaction temperature are trapped into an ice-cooling trap 8, and tetralin is used as an internal standard material. Then, by means of a FID-TCD integrated gas chromatograph (Shimadzu GC-2014), unreacted ethylbenzene and main product styrene, by-product benzene and toluene are quantitatively determined, and an ethylbenzene conversion is calculated based on the following [Formula 5].
[Formula 5]
$$\text{Ethylbenzene Conversion }(\%) = (\text{Sty}+\text{Bz}+\text{Tol}) \div (\text{Eb}+\text{Sty}+\text{Bz}+\text{Tol}) \times 100$$

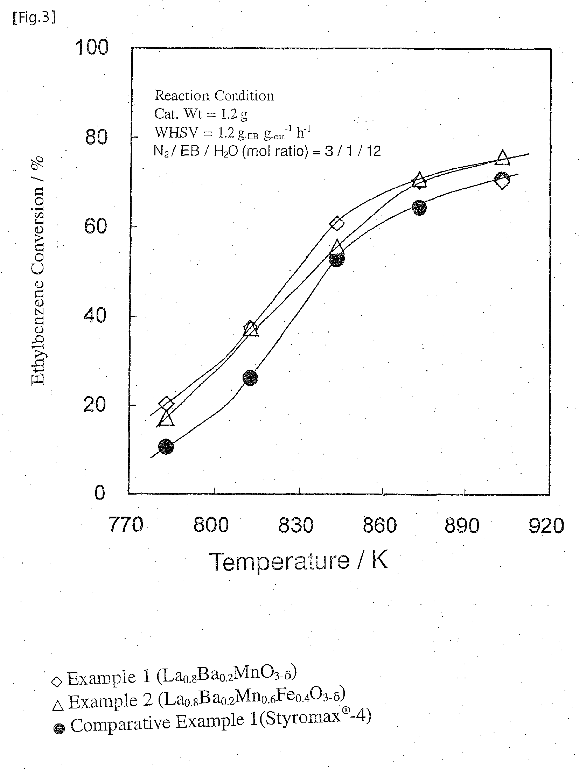

Where
EB: amount of unreacted ethylbenzene/mol
Sty: amount of produced styrene/mol
Bz: amount of produced benzene/mol
TL: amount of produced toluene/mol.
The ethylbenzene conversion at each reaction temperature is shown in Fig.3.

Measurement of Redox Ability

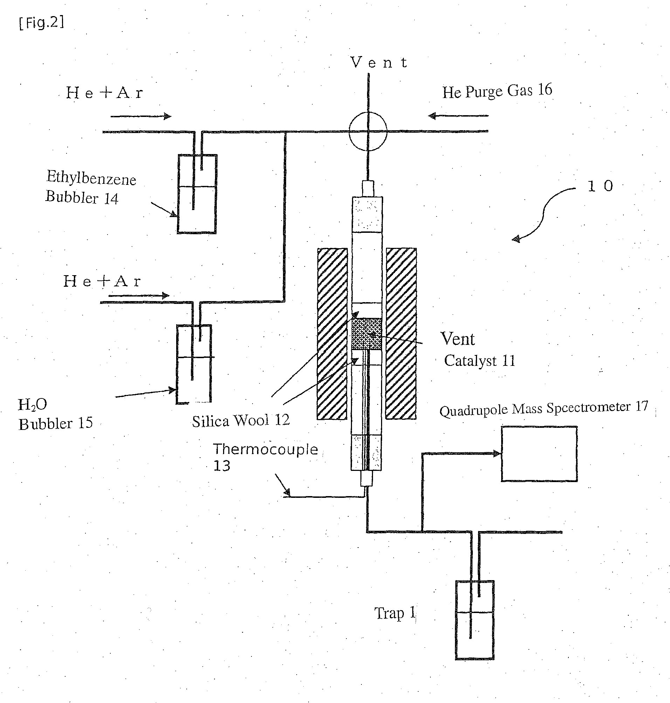

To investigate the redox ability, ethylbenzene and H$_2$O are independently periodically and alternatively passed by means of the measurement device for redox transient response as shown in Fig.2, and the lattice oxygen atomic weight utilizable for the redox reaction in the catalyst is measured. The measurement is carried out as follows.

(Pretreatment)

0.10 g of the catalyst 11 is arranged to a soaking part of the reaction tube and is fixed by arrange a silica wool 12 above and under it, and then temperature is raised to 813 K at the rate of 10 K · min$^{-1}$ under a He stream. Subsequently, a He+Ar gas is passed through a ethylbenzene bubbler 14 and a H$_2$O bubbler 15, a mixture gas with ethylbenzene/H$_2$O/He/Ar=1/12/284/3 ml • min$^{-1}$ is contacted with the catalyst at 813 K, and the ethylbenzene dehydrogenation reaction is carried out. Until the conversion is constant, the mixture gas is flowed, and then the feeding of the He+Ar gas is stopped to flow a He purge gas 16, and the pretreatment is complete.

(Measuring the amount of the discharged lattice oxygen)

To investigate the amount of the discharged lattice oxygen by oxidatively dehydrogenate ethylbenzene indicated in [Formula 1], the He+Ar gas is passed through the ethylbenzene bubbler 14, an ethylbenzene mixture gas with ethylbenzene/He/Ar=1/296/3 ml • min$^{-1}$ is passed through the catalyst 10 for 5 minutes, and a temporal change of the reaction effluence is determined by means of a quadrupole mass spectrometer 17. The amount of $H_2O$ produced by oxidatively dehydrogenate ethylbenzen is quantitatively determined, and the amount of the discharged lattice oxygen of the catalyst ($\mu$mol • g-$_{cat}$) is obtained.

(Measuring the amount of the regenerated oxygen defect in the catalyst lattice)

After contacting ethylbenzene with a mixture gas of noble gases, a He purge gas 16 is flowed. To investigate the amount of the regenerated oxygen defect in the catalyst lattice by steam indicated in [Formula 2], the He+Ar gas is passed through the steam bubbler, a mixture gas with $H_2O$/He/Ar=12/285/3 ml • min$^{-1}$ is passed through for 5 minutes, and a temporal change of the reaction product is determined by means of the quadrupole mass spectrometer 17. The amount of $H_2$ produced by contacting steam is quantitatively determined, and the amount of the regenerated oxygen defect in the catalyst lattice ($\mu$mol • g-$_{cat}$) is measured.
The above-mentioned "measuring the amount of the discharged lattice oxygen" and "measuring the amount of the regenerated oxygen defect in the catalyst lattice" is alternatively carried out three times, and the average is obtained by each measurement. The obtained amount of oxygen utilizable for redox reaction, that is, the amount of discharged lattice oxygen or the amount of regenerated oxygen defect in the catalyst lattice is divided by an "amount of all lattice oxygen in the catalyst" calculated by [Formula 4], and the obtained ratio is listed in Table 1 as below.

[Example 2]

The catalyst performance and redox ability are measured by means of the method as described in Example 1, except for using 7.161 g of La(NO$_3$)$_3$ • 6H$_2$O, 1.091 g of Ba(NO$_3$)$_2$, 3.362 g of Mn(NO$_3$)$_3$ • 6H$_2$O and 3.337 g of Fe(NO$_3$)$_3$ • 9H$_2$O to prepare the catalyst with the composition of La$_{0.8}$Ba$_{0.2}$Mn$_{0.6}$Fe$_{0.4}$O$_{3-\delta}$. The ethylbenzene conversion is shown in Fig.3, and the redox ability is shown in Table 1.

[Comparative Example 1]

The activity test as shown in Example 1 is carried out by means of the catalyst in which the ethylbenzene dehydrogenation catalyst Styromax®-4 (Sued-Chemie Catalysts Japan, Inc) having Fe-K as a main component is comminuted to granulate to 0.50 to 0.71 mm of a particle size by means of a sieve. Before the activity test, an activation treatment is carried out for 3 hours at a temperature of 873 K, a $H_2O$/ $H_2$ molar ratio=12, an entire gas flow rate of 2.2×10$^{-1}$ mol • h$^{-1}$ to activate the catalyst. The result of activity test is shown in Fig.3.

[Comparative Example 2]

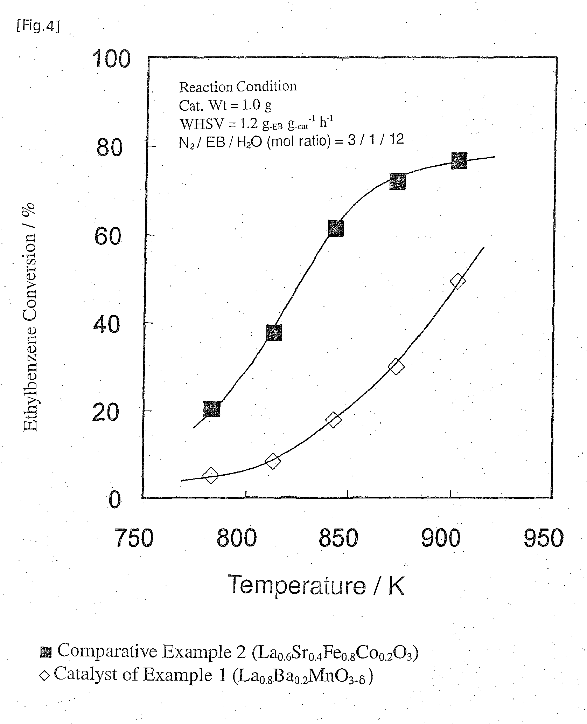

With reference to the composition as described in Example 2 of WHO-A 99/64377, 5.829 g of La(NO$_3$)$_3$ • 6H$_2$O, 1.939 g of Sr(NO$_3$)$_2$, 7.258 g of Fe(NO$_3$)$_3$ • 9H$_2$O and 1.280 g of Co(NO$_3$)$_2$.6H$_2$O are used to prepare the catalyst with the composition of La$_{0.6}$Sr$_{0.4}$Fe$_{0.8}$Co$_{0.2}$O$_3$, and the perovskite structure is confirmed by means of a XRD measurement. Using the catalyst, the activity test is carried out by means of the method as described in Example 1, except for using the weigh hourly space velocity of 1.0 g-ethylbenzene • g-$_{cat}$$^{-1}$•h$^{-1}$. The result of activity test is shown in Fig.4.

[Example 3]

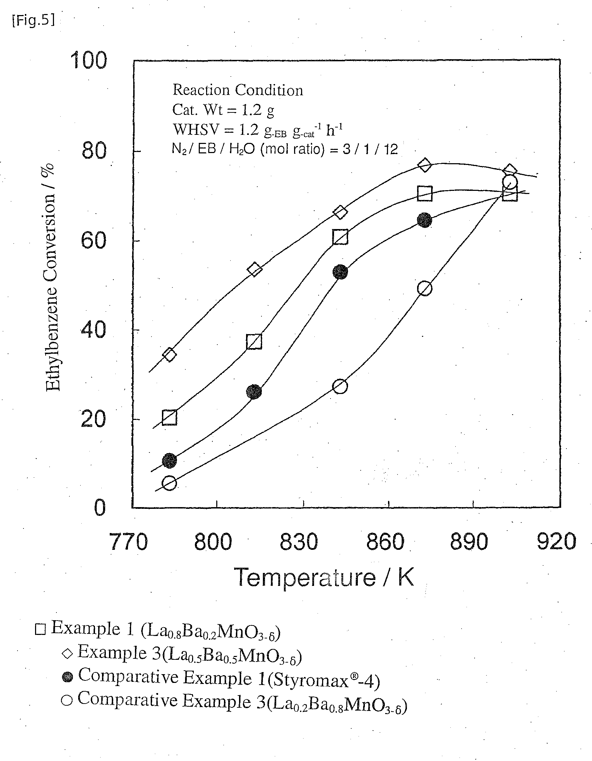

The catalyst performance is measured by means of the method as described in Example 1, except for using 4.492 g of La(NO$_3$)$_3$ • 6H$_2$O, 2.738 g of Ba(NO$_3$)$_2$ and 6.075 g of Mn(NO$_3$)$_3$ • 6H$_2$O to prepare the catalyst with the composition of La$_{0.5}$Ba$_{0.5}$MnO$_{3-\delta}$. The ethylbenzene conversion is shown in Fig.5.

[Comparative Example 3]

The catalyst performance is measured by means of the method as described in Example 1, except for using 4.390g of La(NO$_3$)$_3$ • 6H$_2$O, 4.390 g of Ba(NO$_3$)$_2$ and 6.087 g of Mn(NO$_3$)$_3$ • 6H$_2$O to prepare the catalyst with the composition of La$_{0.2}$Ba$_{0.8}$MnO$_{3-\delta}$. The ethylbenzene conversion is shown in Fig.5.

[Comparative Example 4]

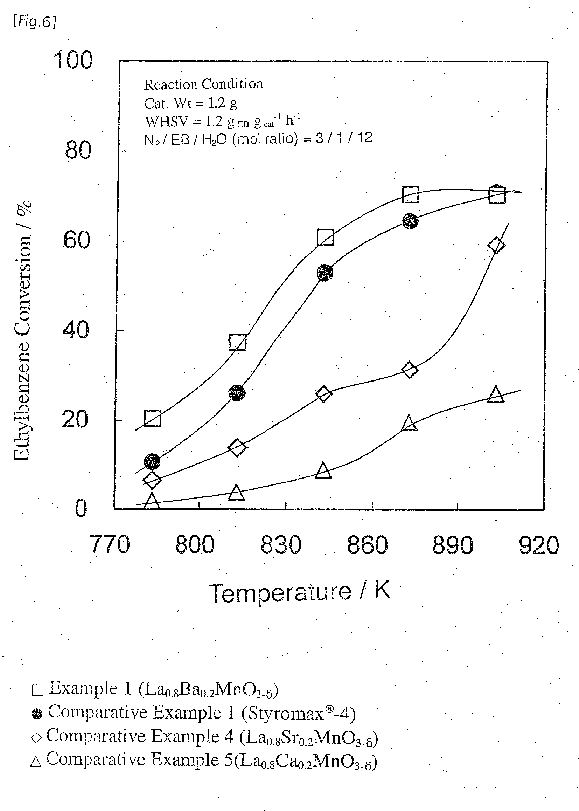

The catalyst is prepared by means of the method as described in Example 1, and its performance and redox ability are measured, except for using 7.483 g of $La(NO_3)_3 \cdot 6H_2O$, 0.933 g of $Sr(NO_3)_2$ and 6.324 g of $Mn(NO_3)3 \cdot 6H_2O$ to prepare the catalyst with the composition of $La_{0.8}Sr_{0.2}MnO_{3-\delta}$. The ethylbenzene conversion is shown in Fig.6, and the redox ability is shown in Table 1.

[Comparative Example 5]

The catalyst is prepared by means of the method as described in Example 1, and its performance and redox ability are measured, except for using 7.800 g of $La(NO_3)_3 \cdot 6H_2O$, 1.080 g of $Ca(NO_3)_2 \cdot 6H_2O$ and 6.594g of $Mn(NO_3)_3 \cdot 6H_2O$ to prepare the catalyst with the composition of $La_{0.8}Ca_{0.2}MnO_{3-\delta}$. The ethylbenzene conversion is shown in Fig. 6, and the redox ability is shown in Table 1.

[Industrial Applicability]

As the dehydrogenation catalyst for producing alkenyl aromatic compounds by means of steam, the catalyst focused on having the high redox ability is produced. Thus, it is possible to provide the catalyst which has the same yield as the catalyst containing the conventionally necessary alkali metal component and which considerably improves the operation problems caused by the alkali metal component migrated from the catalyst during the reaction, and the process for producing same, and the dehydrogenation method using it. Therefore, the very advantage productivity is created in the catalyst field.

[Table 1]

| | Composition of catalyst | Amount of all lattice oxygen [1] /$\mu$mol $\cdot$ $g_{-cat}^{-1}$ | Amount of discharged lattice oxygen /$\mu$mol $\cdot$ $g_{-cat}^{-1}$ | Amount of regenerated lattice oxygen defect /$\mu$mol$\cdot g_{-cat}^{-1}$ | Lattice oxygen utilizable for redox reaction to all lattice oxygen /% | |
|---|---|---|---|---|---|---|
| Example 1 | $La_{0.8}Ba_{0.2}MnO_{3-\delta}$ | $1.24 \times 10^4$ | 502 | 451 | 3.71 | 3.34 |
| Example 2 | $La_{0.8}Ba_{0.2}Mn_{0.6}Fe_{0.4}O_{3-\delta}$ | $1.24 \times 10^4$ | 341 | 323 | 2.52 | 2.39 |
| Example 4 | $La_{0.8}Sr_{0.2}Mn_4O_{3-\delta}$ | $1.30 \times 10^4$ | 299 | 297 | 2.19 | 2.19 |
| Example 5 | $La_{0.8}Ca_{0.2}Mn_4O_{3-\delta}$ | $1.35 \times 10^4$ | 134 | 136 | 0.99 | 0.99 |

[1] Amount of all lattice oxygen ($\mu$mol $\cdot$ $g_{-cat}^{-1}$) = $3 \times 10^6 \div$ formula mass of each catalyst
[2] ratio of amount of utilizable lattice oxygen (%) = (amount of discharged lattice oxygen) $\div$ (amount of all lattice oxygen)
[3] ratio of amount of utilizable lattice oxygen (%) = (amount of regenerated lattice oxygen) $\div$ (amount of all lattice oxygen)

**Claims**

1.  A dehydrogenation catalyst of alkylaromatic compounds for producing alkenylaromatic compounds by means of dehydrogenation by contacting alkyl aromatic compounds diluted with steam, wherein the catalyst contains oxides having redox ability.

2.  The dehydrogenation catalyst of alkylaromatic compounds of claim 1 wherein the alkylaromatic compound is ethylbenzene and the alkenyl aromatic compound is styrene monomer.

3.  The dehydrogenation catalyst of alkyl aromatic compounds of claim 1 wherein:

redox ability is indicated by an amount of oxygen utilizable for redox reactions in a lattice oxygen in the catalyst; and

an amount of catalytic lattice oxygen utilizable for redox reactions measured by a redox transient response method is greater than or equal to 2.4 % of total amount of the lattice oxygen in the catalyst.

4. The dehydrogenation catalyst of alkyl aromatic compounds of claim 3 wherein the oxide is a mixed metal oxide containing rare-earth metal, alkaline-earth metal and transition metal but not containing alkali metal.

5. The dehydrogenation catalyst of alkyl aromatic compounds of claim 4 containing a mixed metal oxide comprising any one of La or Ce of rare-earth metal, one or more elements selected from the group of alkaline-earth metals consisting of Mg, Ca, Sr and Ba and one ore more elements selected from the group of transition metals consisting of Ti, Cr, Mn, Fe, Co, Ni and Cu.

6. The dehydrogenation catalyst of alkylaromatic compounds of claim 4 wherein the mixed metal oxide comprises a group of elements consisting of La, Ba, Mn and Fe.

7. The dehydrogenation catalyst of alkylaromatic compounds of claim 4 wherein the mixed metal oxide has a spinel structure or a perovskite structure.

8. A process for producing a calcined dehydrogenation catalyst of alkylaromatic compounds comprising the following steps of;

    - preparing an extrudable mixture by admixing the compositions of the dehydrogenation catalyst of alkyl aromatic compounds of claim 1 with sufficient water to form an extrudable mixture;
    - molding the extrudable mixture into a pellet; and
    - drying and then calcining the pellet to form a finished catalyst.

9. A method for dehydrogenation of alkylaromatic compounds to produce alkenylaromatic compounds by contacting alkylaromatic compounds with steam in the presence of the dehydrogenation catalyst of alkylaromatic compounds of claim 1.

[Fig.1]

[Fig.2]

Vent

He+Ar

He Purge Gas 16

Ethylbenzene
Bubbler 14

10

He+Ar

Vent
Catalyst 11

H₂O
Bubbler 15

Silica Wool 12

Quadrupole Mass Spcectrometer 17

Thermocouple
13

Trap 1

[Fig.3]

Reaction Condition
Cat. Wt = 1.2 g
WHSV = 1.2 $g_{EB}$ $g_{cat}^{-1}$ $h^{-1}$
$N_2$ / EB / $H_2O$ (mol ratio) = 3 / 1 / 12

◇ Example 1 ($La_{0.8}Ba_{0.2}MnO_{3-\delta}$)
△ Example 2 ($La_{0.8}Ba_{0.2}Mn_{0.6}Fe_{0.4}O_{3-\delta}$)
● Comparative Example 1(Styromax®-4)

[Fig.4]

Comparative Example 2 ($La_{0.6}Sr_{0.4}Fe_{0.8}Co_{0.2}O_3$)
◇ Catalyst of Example 1 ($La_{0.8}Ba_{0.2}MnO_{3-\delta}$)

[Fig.5]

Reaction Condition
Cat. Wt = 1.2 g
WHSV = 1.2 $g_{-EB}$ $g_{-cat}^{-1}$ $h^{-1}$
$N_2$ / EB / $H_2O$ (mol ratio) = 3 / 1 / 12

□ Example 1 ($La_{0.8}Ba_{0.2}MnO_{3-\delta}$)
◇ Example 3($La_{0.5}Ba_{0.5}MnO_{3-\delta}$)
● Comparative Example 1(Styromax®-4)
○ Comparative Example 3($La_{0.2}Ba_{0.8}MnO_{3-\delta}$)

[Fig.6]

□ Example 1 (La$_{0.8}$Ba$_{0.2}$MnO$_{3-\delta}$)

● Comparative Example 1 (Styromax®-4)

◇ Comparative Example 4 (La$_{0.8}$Sr$_{0.2}$MnO$_{3-\delta}$)

△ Comparative Example 5(La$_{0.8}$Ca$_{0.2}$MnO$_{3-\delta}$)

## INTERNATIONAL SEARCH REPORT

| | International application No. |
|---|---|
| | PCT/JP2008/067134 |

| A. CLASSIFICATION OF SUBJECT MATTER |
|---|
| B01J23/34(2006.01)i, B01J23/889(2006.01)i |

According to International Patent Classification (IPC) or to both national classification and IPC

| B. FIELDS SEARCHED |
|---|
| Minimum documentation searched (classification system followed by classification symbols) |
| B01J23/34, B01J23/889 |

| Documentation searched other than minimum documentation to the extent that such documents are included in the fields searched |
|---|
| Jitsuyo Shinan Koho        1922-1996   Jitsuyo Shinan Toroku Koho    1996-2008 |
| Kokai Jitsuyo Shinan Koho   1971-2008   Toroku Jitsuyo Shinan Koho    1994-2008 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y | JP 11-104494 A  (Sud Chemie Mt S.r.l.),<br>20 April, 1999 (20.04.99),<br>Claims; Par. Nos. [0023] to [0031]<br>& US 6184174 B1        & EP 894528 A2 | 1-3,8-9<br>4-7 |
| X<br>Y | JP 10-359 A  (Montecatini Technologies S.r.l.),<br>06 January, 1998 (06.01.98),<br>Claims; Par. Nos. [0034] to [0043]<br>& US 6166280 A          & EP 794004 A1 | 1-3,8-9<br>4-7 |
| X<br>Y | JP 5-68885 A  (Nissan Girdler Catalyst Co.,<br>Ltd.),<br>23 March, 1993 (23.03.93),<br>Claims; Par. Nos. [0001], [0034] to [0046]<br>& US 5190906 A          & EP 502510 A1 | 1-3,8-9<br>4-7 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 November, 2008 (28.11.08) | 09 December, 2008 (09.12.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

| International application No. |
|---|
| PCT/JP2008/067134 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JP 2000-505771 A  (Rhodia Chimie),<br>16 May, 2000 (16.05.00),<br>Abstract; Claims; pages 4 to 6, 13 to 18<br>& WO 1998/024726 A1     & US 2002/0115563 A1<br>& EP 950022 A | 1-3,8-9<br>4-7 |
| Y | JP 2007-224747 A  (Mitsubishi Motors Corp.),<br>06 September, 2007 (06.09.07),<br>Claims; Par. Nos. [0017] to [0021], [0043] to<br>[0055]<br>& US 2007/0196246 A1     & EP 1820562 A1 | 4-7 |
| Y | JP 9-86928 A  (Nissan Motor Co., Ltd.),<br>31 March, 1997 (31.03.97),<br>Claims; Par. Nos. [0011] to [0012], [0037] to<br>[0096], [0124] to [0129]<br>& US 6060420 A          & US 6129862 A | 4-7 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 3881376 A
- WO 2833907 A
- WO 2604426 A
- WO 9964377 A

**Non-patent literature cited in the description**

- *Catalyst,* 1991, vol. 33 (1), 9-14
- *Applied Catalysis,* 1986, vol. 26, 81
- *Journal of Catalysis,* 1990, vol. 126, 339